# EUROPEAN PATENT APPLICATION

(11) **EP 1 046 710 A1**
(43) Date of publication of application: **25.10.2000**
(21) Application number: 99108068.0
(22) Date of filing: 23.04.1999
(51) Int. Cl.: C12N 15/85, C12N 15/63, C12N 15/62, C07K 14/47, C07K 14/705

(54) **Promoter-transactivator system for inducible high-level mammalian gene expression with the option of cell growth control**

(71) Applicant: Gesellschaft für Biotechnologische Forschung mbH (GBF), 38124 Braunschweig (DE)
(72) Inventor: Mueller, Peter P., 38124 Braunschweig (DE); Geserick, Christoph, 38124 Braunschweig (DE); Schroeder, Katharina, 38124 Braunschweig (DE); Hauser, Hansjoerg, 38124 Braunschweig (DE)
(74) Representative: Bauer, Robert, Dipl.-Ing.

(57) **Abstract**

A promoter-transactivator system achieves regulated high-level gene expression in proliferation-controlled mammalian cells. The novel composite promoter contains constitutive enhancer elements that allows a basal expression level as high as the levels achieved with a very efficient viral promoter. In addition, the promoter encodes sequences that are bound by a transactivator whose activity can be regulated. By the simple addition of medium supplements expression levels can be achieved above those from the conventional promoter.

### Annex to the application documents - subsequently filed sequences listing

SEQUENCE LISTING

<110> Gesellschaft für Biotechnologische Forschung mbH

<120> Promoter-transactivator system for inducible high-level
      mammalian gene expression with the option of cell
      growth control

<130> EP 99 108 068.0

<140> 99 108 068.0
<141> 1999-04-23

<160> 3

<170> PatentIn Ver. 2.1

<210> 1
<211> 270
<212> DNA
<213> Myeloproliferative Sarcoma Virus

<400> 1
gctagcttaa gtaacgccat tttgcaaggc atgggaaaaa tacataactg agaatagaga 60
agttcagatc aaggtcagga acagagaaac aggagaatat gggccaaaca ggatatctgt 120
ggtaagcagt tcctgccccg ctcagggcca agaacagttg gaacaggaga attgggccaa 180
acaggatatc tgtggtaagc agttcctgcc ccgctcaggg ccaagaacag atggtcccca 240
gatgcggtcc cgccctcagc agtttctaga                                  270

<210> 2
<211> 70
<212> DNA
<213> Firefly

<400> 2
gatcccttct cgggaaatgg aaactgaaaa tcagatccct tctcgggaaa tggaaactga 60
aaatcagatc                                                        70

<210> 3
<211> 74
<212> DNA
<213> Cytomegalo Virus

<400> 3
tggcgtgtac ggtgggaggc ctatataagc agagctcgtt tagtgaaccg tcaaaccgtc 60
aaaccgcgga agct                                                   74

## Description

### Background of invention

The genetic modification of mammalian cells to express recombinant products is a key strategy in research and biotechnological applications, and mammalian cell cultures have become the preferred production system for secreted pharmaceutical proteins (Hauser, 1997; for complete bibliography of citations see reference list at the end of this specification).

**Proliferation control:** In the natural environment, cell growth is strictly regulated. Life of a mammal begins with fertilization of the oocyte that divides and proliferates until the animal reaches its mature size. In the adult, proliferation and cell death are balanced to keep the total cell mass essentially constant, while the synthesis and secretion of cellular products continues.

In contrast to the natural situation, due to the requirement to obtain large numbers of cells needed in production processes, mammalian producer cell lines have been selected for rapid and indefinite proliferation capacity. Cell lines used in industrial production processes are transformed and override natural growth control systems such as contact inhibition, anchorage-dependent growth, naturally limited numbers of cell divisions, organ size control and others. Cell growth is a requirement for the genetic manipulation and cloning of cells and then to achieve a sufficient number of producer cells from a single cell initially. However, once a high cell density is reached in a production process, further cell proliferation is associated with disadvantages such as genetic instability, medium depletion, accumulation of toxic products, followed by cell death and cell lysis that leads clogging of cell retention and product purification devices, product contamination with cellular debris and product deterioration due to glycosidases and proteases (Fussenegger et al., 1999).

Regulated cell growth could mimic the natural situation in technical applications, with rapid proliferation initially until they reach an optimal cell density. Then reduced growth would extend the productive period and keep production conditions constant by lowering medium consumption and waste product accumulation. Since fewer resources are consumed for the synthesis of cell mass, production would be more efficient. The reduced cell division rate would also reduce genetic drift, and by that stabilize the productivity. Therefore, growth regulation could increase production, product quality and consistency.

Genetic growth control systems have specific advantages. There is no need to change production conditions by the addition of toxic compounds, nutrient limitations or suboptimal temperatures that may affect productivity or product quality. The genetic systems are flexible and allow the stepwise improvement of the recombinant regulatory system, optimization of the producer cell and the production conditions. An additional advantage is that the expression of recombinant genes can be induced during growth arrest by using dedicated promoters. Specific requirements must be met by growth control systems concerning stability, productivity, cell viability, quality and consistency of the product and applicability to industrial fermenter systems. Presently under investigation are a system based on cell cycle inhibitory protein overexpression (Fussenegger et al., 1998; Fussenegger et al., 1997; Mazur et al., 1998) and a system using c-jun antisense RNA expression (Kim et al., 1998).

**Stabilization of gene expression:** The isolation of stable clones is currently a major obstacle for recombinant gene expression in mammalian cells. To date, there is no reliable method that can be afforded by the average research laboratory. In general, in addition to a gene of interest to be expressed, a marker gene is introduced that facilitates the isolation of producer cell clones. However, not all cells that express the marker gene do also express the gene of interest, and furthermore, the expression-level of the two genes do not necessarily correlate in individual cells, this is a considerable problem especially for the selection of highest-level producer clones and for the selection of cell clones that for an extended time period are to express a protein of interest that has a negative effect on cell growth or viability. The correlation of expression-levels of two genes that are introduced simultaneously depends on the strategy used. The simplest procedure with least tight coupling is the cotransfection method whereby the genes are encoded on different DNA molecules. Tighter coupling is achieved when different genes are coexpressed either from a bi-directional promoter or, preferably, as a polycistronic RNA. Tight coupling of a gene of interest with a selection marker gene ensures stable expression of both genes under selective conditions. This is especially important when the gene of interest imposes a negative selection pressure on the expressing cell, such as proliferation inhibitory genes.

**Recombinant gene expression:** Often maximizing production is a costly and time-consuming procedure. A most critical factor for productivity is the recombinant gene promoter activity. For constitutive gene expression, a highly active recombinant promoters are available from a variety of sources. These promoters have been derived from cellular and viral genes, from homologous and heterologous systems or they may contain optimized sequences of non-natural origin. For many applications constitutive expression is neither necessary nor desirable. For example in a biotechnological production process, recombinant gene expression in the initial phases of a fermented run may be a metabolic burden on the producer cells, leading to a negative selection pressure of the most productive cells and decrease the performance of the system, while at low cell densities the contribution to the overall production is negligible. Furthermore, product synthesized early may differ in quality from the bulk product synthesized at high cell densities. Glycoprotein quality depends on the environment of the cells. Since the media compositions, in particular ammonia and glucose concentration, change during the course of fermentations, product glycan structures synthesized early and late in the process differ as well. In addition, secreted products are subject to degradation processes. A long exposure time to degrading enzymes of product synthesized early lead to a lower quality and homogeneity of the product. Therefore, for some applications a system for regulated recombinant gene expression is of advantage. With the exception of the production of highly cytotoxic products, a very high induction rate is not necessary to achieve a higher product quality homogeneity. Despite these advantages, the drawback of regulated gene expression is the generally far less efficient expression compared to the best constitutively active promoters, and a high maximal expression level is of primary interest.

### State of the art:

### Proliferation control:

To regulate cell growth, IRF-1 (Interferon Regulatory Factor 1) constructs were expressed in mammalian cells. IRF-1 is a transcriptional activator of genes which lead to growth inhibition. To control recombinant IRF-1 activity in vivo, two systems were established: A tetracycline dependent IRF-1 transcription system and a regulatable IRF-1-hER (estrogen receptor fusion protein; Koster et al., 1995; Kirchhoff et al., 1996). The addition of 13-estradiol or other estrogen receptor ligands to the growth medium activates IRF-1-hER, leading to transcriptional activation from promoters containing IRF-1-binding sites. The expression level depends on the β-estradiol concentration in the medium and on the duration of the exposure to the hormone (Kirchhoff et al., 1996; Köster et al., 1995). Constitutive expression of IRF-1-hER fusion protein has no effect on cell growth in the absence of the ligand estradiol, and no effect of estradiol on BHK cell growth has been observed in the absence of IRF-1-hER. This proliferation control system has been developed to adjust the cell density of mammalian producer cell cultures (Fussenegger et al., 1999).

**Promoters for recombinant gene expression in mammalian cells:** Presently two kinds of promoter systems are used for efficient recombinant gene expression. For unregulated expression constitutive promoters are either derived from viral promoters of from strong cellular promoters. For regulated gene expression, tetracycline regulated promoter-transactivator systems have become widely used. This system allows very low basal expression and results in high induced expression levels in many cell lines. However, when compared to strong constitutive promoters, the tetracycline system is not for all cell lines an optimal choice to obtain maximal expression levels.

In one aspect the present invention provides a promoter-transactivator system for inducible high-level mammalian cell gene expression with the option of cell growth control as defined in claim 1.

In a further aspect the present invention provides an expression vector(s) comprising a promoter construct and/or a transactivator construct as defined in claim 1 (i. e. said promotor construct and said transactivator construct may be incorporated in the same expression vector thus containing said a promoter-transactivator system or may be incorporated in separt expression vectors).

In another aspect the present invention provides mammalian cells transfected or transformed with said expression vector(s).

In yet another aspect the present invention provides a process for inducible high-level mammalian gene expression with the option of cell growth control as defined in claim 4.

The invention is now illustrated in more detail.

A system was established that allows regulated high-level gene expression in mammalian cells. It consists of a regulated transcriptional activator IRF-1 and a inducible promoter for high-level recombinant gene expression.

BHK-21 cells were genetically engineered to express regulatable IRF-1 fusion proteins. The inventors investigated several properties that are important to control cell growth in production processes. The regulation was stabilized by using a dicistronic construct to couple the transcription of the IRF-1 gene to an antibiotic resistance selection marker gene. Permanent IRF-1 activation for longer than two to four days lead to viability decreases. To maintain a high cell viability, a schedule was developed of IRF-1 activation and inactivation in several cycles to control cell growth over a period of at least one month while maintaining a high cell viability. Since IRF-1 is a transcriptional activator, promoters containing IRF-1 binding sites can be employed to increase transcription to levels higher than those achieved with strong viral promoters. When IRF-1 is activated, the glycan structure of a relevant pharmaceutical product remained essentially the same and there was no influence on site occupancy or protein integrity.

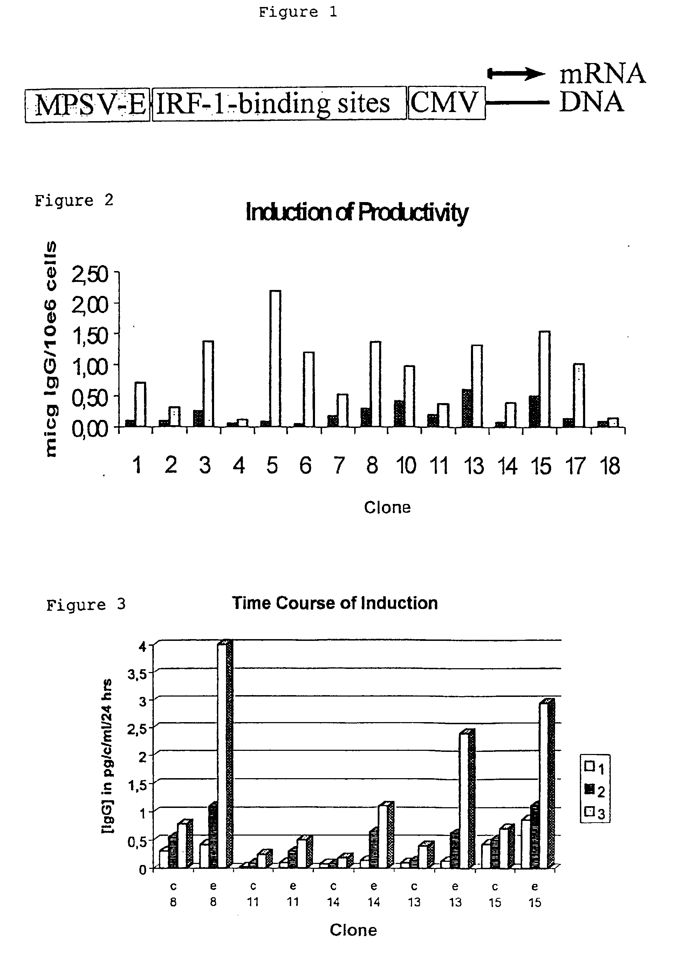

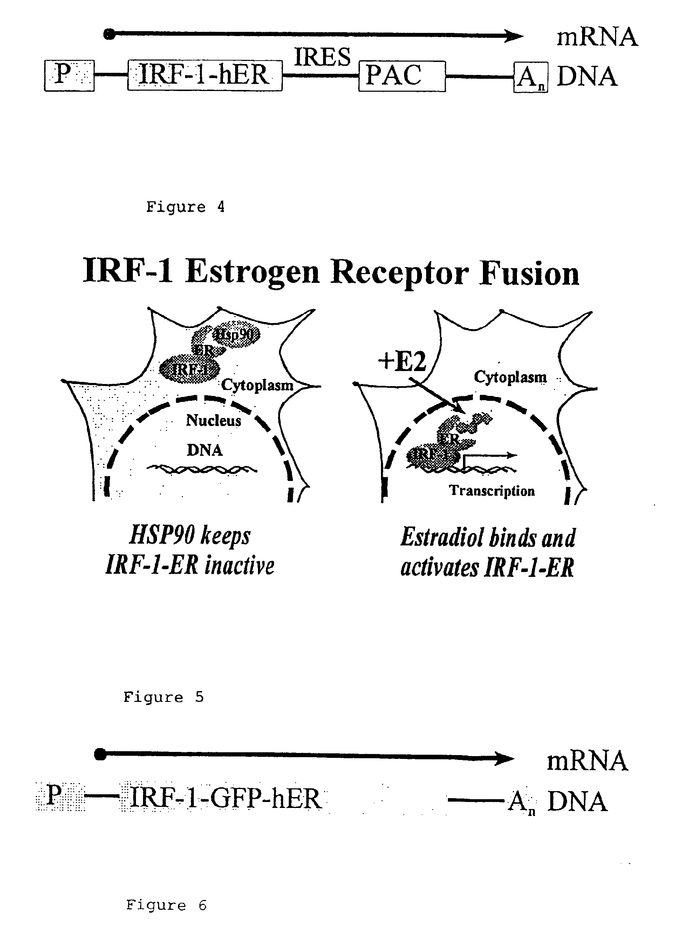

The present invention is related to:
1) A regulated promoter that shows a higher maximal secreted pharmaceutical protein productivity than a contemporary highly efficient viral promoter (MPSV, myeoloproliferative sarcoma virus). The artificial promoter consists of viral enhancer repeats (MPSV), of IRF-1-binding sites (ISRE-luc, Interferon Stimulated Response Element-firefly luciferase: Kirchhoff and Hauser, 1999) and of a viral minimal promoter element (CMV, cytomegalo virus); (Table 1, Figure 1 and 2).
2) BHK-21 cell lines that expresses an IRF-1-human estrogen receptor (hER) construct whose expression is stabilized by a dicistronic expression construct with a drug resistance gene (puromycin acetyltransferase) as a selection marker (Figure 3).
3) An IRF-1-GFP-hER fusion that can be used for FACS analysis and sorting of transfected cells (Figure 4). The IRF-hER fusion protein activates promoters with IRF-1-binding sites (for a simplified model see figure 5).

An advantage of the present invention is that secreted recombinant glycoprotein protein quality is maintained in IRF-1 expressing cells (Figure 6).

The promoter (hereinafter also referred to as IRFE promoter) for inducible high-level mammalian gene expression has the composition of Figure 1, wherein
**MPSV-E** means MPSV enhancer repeats, e. g. from pMBC-lNheI-XhoI, of the sequence: 
GCTAGCTTAAGTAACGCCATTTTGCAAGGCATGGAAAAATACATAACTGAGAATAGAGAAG
TTCAGATCAAGGTCAGGAACAGAGAAACAGGAGAATATGGGCCAAACAGGATATCTGTGGTA
AGCAGTTCCTGCCCCGCTCAGGGCCAAGAACAGTTGGAACAGGAGAATTGGGCCAAACAGGA
TATCTGTGGTAAGCAGTTCCTGCCCCGCTCAGGGCCAAGAACAGATGGTCCCCAGATGCGGT
CCCGCCCTCAGCAGTTTCTAGA,
 or isofunctional (i. e. having the same biological activity or function) variants thereof obtained by substitution, insertion or deletion of one or more nucleotides,
**IRF-1 binding sites** means the sequence: 
GATCCCTTCTCGGGAAATGGAAACTGAAAATCAGATCCCTTCTCGGGAAATGGAAACTGAAA
ATCAGATC,
 or isofunctional variants thereof obtained by substitution, insertion or deletion of one or more nucleotides,
and **CMV** means the CMV minimal promoter, e. g. from the plasmid pGL2 (Promega, Madison, WI): 
TGGCGTGTACGGTGGGAGGCCTATATAAGCAGAGCTCGTTTAGTGAACCGTCAAACCGTCAA
ACCGCGGAAGCT,
 or isofunctional variants thereof obtained by substitution, insertion or deletion of one or more nucleotides,

The composite promoter for inducible high-level expression as depicted in the above Figure 1 thus consists of constitutive viral enhancer elements derived from MPSV, IRF-1 binding sequences and a minimal promoter sequence from CMV that determines the RNA start site.

It is appreciated that the above enhancer elements, binding sequences and promoter sequence may be separated by linker or spacer sequences.

### Examples and comparative examples

Table 1 shows the basal level expression from the inventive IRFE promoter and a viral promoter

**Table 1**

| Promoter | IgG [mg/ml] |
|---|---|
| IRFE | 1.8 +/- 0.1 |
| IRFE + MPSV | 1.7 +/- 0.1 |
| MPSV | 1.7 +/- 0.1 |

From Table 1 it can be seen that the basal level expression from the IRFE promoter is as efficient as a highly active viral promoter. Adherent BHK-21 cells grown in DME medium with 10% FCS were transiently transfected by the calcium cotransfection method with 1 microgram plasmid DNA per ml of culture medium. A sample from the medium supernatant was removed after 24 hrs and the antibody concentration determined by a sandwich ELISA in 96 well microtiter plates. For coating, anti-human IgG (Fab specific) goat antibody (Sigma Immuno Chemicals, No I-5260) was used at a 1:1500-fold dilution. Detection was by a 1:5000 diluted peroxydase conjugated affinity purified goat anti human IgG (H+L) (Jackson ImmunoResearch Laboratories , Dianova, Hamburg, number 109-035-088) followed by a colorimetric peroxydase assay using ortho-phenyldiamine. Human IgG1 Kappa (Sigma, number I-3889) was used as a standard. The absorption at 490 nm was measured in an automatic microplate reader (Cytofluor 2300, Millipore). MPSV: A dicistronic construct with the polio virus IRES element (Sonenberg 1990) expressing recombinant human IgG anti CMV antibody heavy and light chain from the myeloproliferative sarcoma virus promoter (Boldicke et al., 1995; Dirks et al., 1994; Dirks et al., 1993; Arelt et al., 1988). IRFE: the same dicistronic IgG antibody expressed from the IRF-1-inducible promoter. IRFE +MPSV: DNA from both plasmids were mixed at a final concentration of .5 microgram plasmid DNA each per ml of culture medium.

Figure 2 shows the enhanced productivity from the IRFE promoter in IRF-1 induced cells. IRF-1-hER expressing BHK-21 cells were transfected with DNA encoding an IgG antibody gene that is transcribed from the IRFE promoter. 15 IgG producer clones were identified by a filter immunoassay (Wirth et al., 1990). Induced IgG expression was determined after 48 hours in the presence of 100 nM estradiol (right bars) or for basal level expression in the absence of estradiol (left bars). IgG concentration in the supernatant was determined by a sandwich ELISA as described in Table 1.

Figure 3 shows that IgG productivity increases gradually after induction. BHK-21 cell clones transfected with IRF-1-hER and IRFE-IgG. IgG expression from an IRF-1 responsive promoter was determined without (c) and with estradiol present in the medium (e) after 1, 2 or 3 days.

Figure 4 shows a IRF-1-hER expression construct . IRF-1-hER is stably expressed from a constitutive viral promoter (P; Dirks et al., 1994) on a dicistronic mRNA together with puromycin transacetylase as a selection marker gene (PAC; Vara et al., 1986) that is translated due to the presence of a polioviral internal ribosomal entry site (IRES; Sonenberg 1990). The SV40 poly A addition site determines the end of the mRNA. This configuration allows to select transfected cells that efficiently express this construct.

Figure 5 shows the IRF-hER (IRF-ER) protein to be a transcriptional activator that is regulated by ligands such as estradiol (E2; right). It binds to DNA sequences that contain IRF-binding sites. In the absence of ligand, the IRF-hER fusion gene has a low activity (left).

Figure 6 shows an IRF-1-GFP hER expression construct. The eGFP (Promega) reading frame has been inserted in frame into a BamHI restriction site between the reading frames for IRF-1 and hER. IRF-1-GFP hER is expressed from a constitutive viral promoter (P; Dirks et al., 1994).

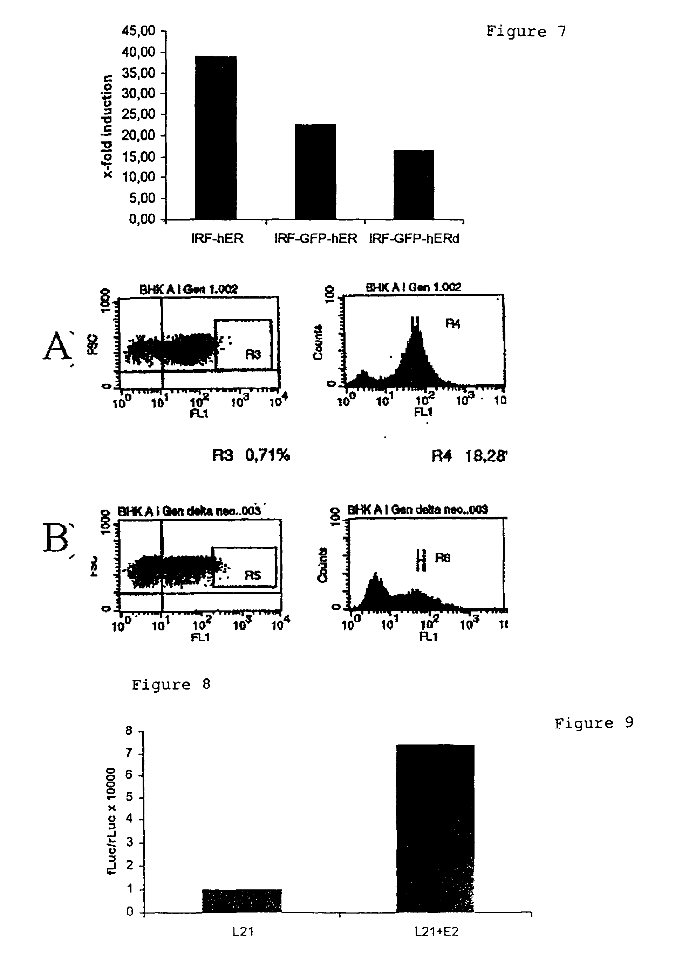

Figure 7 shows the estrogen regulated transcriptional activation of IRF binding sites containing promoters by IRF-GFP-hER expressing cells. NIH3T3 cells were transfected with two different plasmids expressing IRF-GFP-hER that differ by a small deletion (d) outside of the reading frame, and for comparison IRF-hER. Transient luciferase induction from an ISRE promoter element (pGL2-ISRE-luc) was determined in the presence of 1mM estradiol relative to the luciferase activity in the absence of estradiol of Renilla luciferase from a constitutive viral promoter (CMV-rluc).

Figure 8 shows that IRF-1-GFP-hER fluorescence can be used as to isolate fusion gene expressing single cells by FACS sorting. GFP FACS analysis and sorting of BHK-21 cells (A) and BHK-21 cells transfected with IRF-1-GFP hER (B).

Figure 9 shows the estradiol regulated IRF-1-GFP hER activated gene expression from an IRFE promoter. A stably transfected IRF-1-GFP hER expressing L88-5 single cell clone (L21) isolated by FACS sorting of highly fluorescent cells was transiently transfected using Lipofectin (GIBCO/BRL) with a firefly luciferase gene expressed for 16 hours from an IRF1 inducible promoter in the absence (L21) or presence of 1 µM estradiol (L21+E2), relative to constitutively expressed renilla luciferase.

Examples for specific applications in industrial relevant systems.

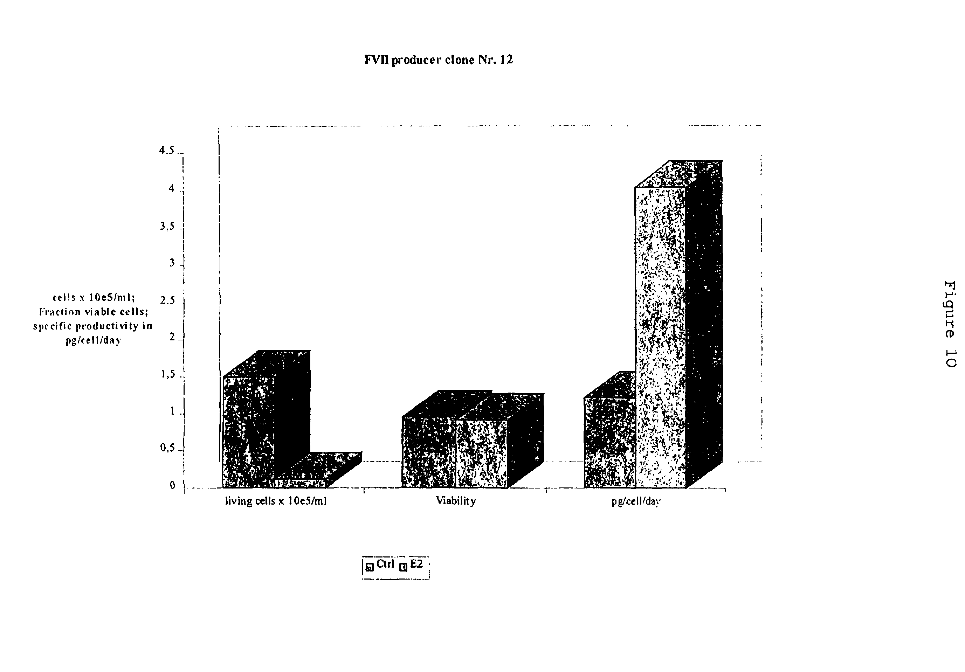

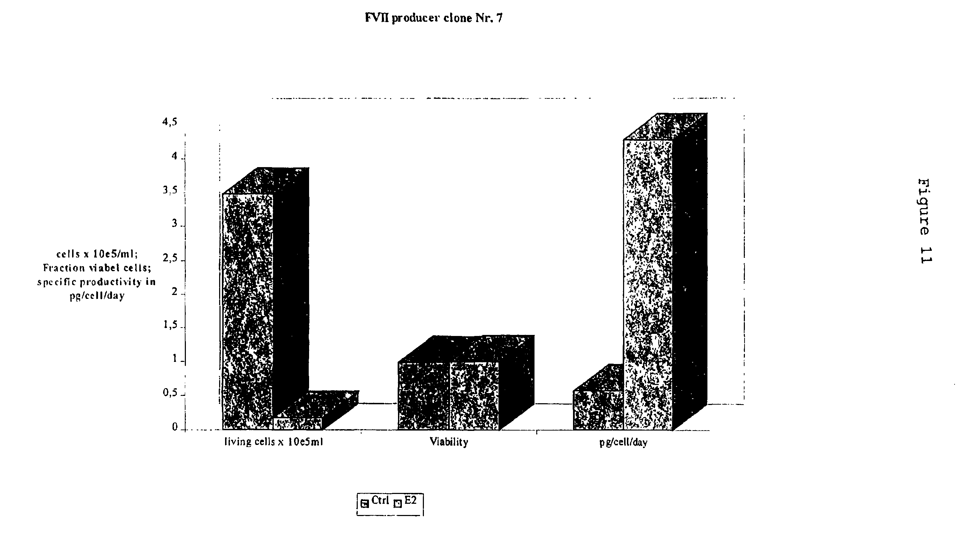

Figures 10 and 11 relate to two examples of a relevant pharmaceutical protein expressing BHK (baby hamster kidney) cell clones with IRF-hER enhanced productivity (pg/cell/day, E2) versus control(pg/cell/day, ctrl) and reduced proliferation (living cells x 10e5/ml, E2) versus controll (living cells, ctrl)

The following is one example for increased IgG antibody productivity in a long-term experiment in IRF-hER proliferation controlled BHK-21 cells in a perfused fermenter with microcarriers showing also high viability. For additional characterization of lesser importance glucose consumption and lactate production was determined.

### Name: Fermenter 3

Conditions: IgG 8 cells, Cytodex 3 microcarrier, 6g/l, medium for all fermenters: DMEM: F12 with 5% serum and 500 µg/ml G418 and 3 µg/ml Puro (with added dextran and Pluronic)

Fermenter: New Brunswick, stirred tank, 5 1 tank capacity, 3.5 1 working volume, settling tube as separator (a glas tube in which the microcarriers settle down by gravitational action) while the medium is removed. Works effective up to a perfusionrate of 1 volume/day (also 3.5 l/day). For higher values gravitational action is not sufficient to compensate sucking action.

At the end of the induction stirring was stopped for 20 min. After the microcarriers have settled down the medium was removed by suction except for 0.5 l, added up to 2 l (=wash), again removed by suction except for 0.5 1 and added up to 3.5 1. Approximatly 7% of the initially content of E2 remains (since, however, during induction perfusion was activated, the actual value for E2 was below 7%). The washing procedure tooks about 1-1.5 h.

In all fermenters the perfusion rate was in the range from 0 to 0.9, wherein most of the time the rate was in the range 0.6 to 0.9.

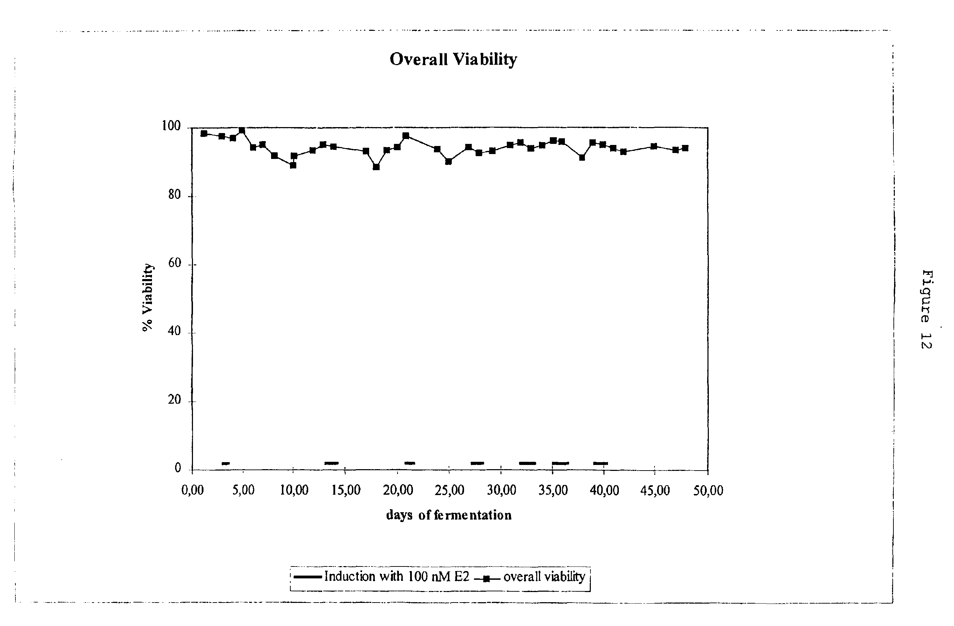

Figure 12 shows the results.

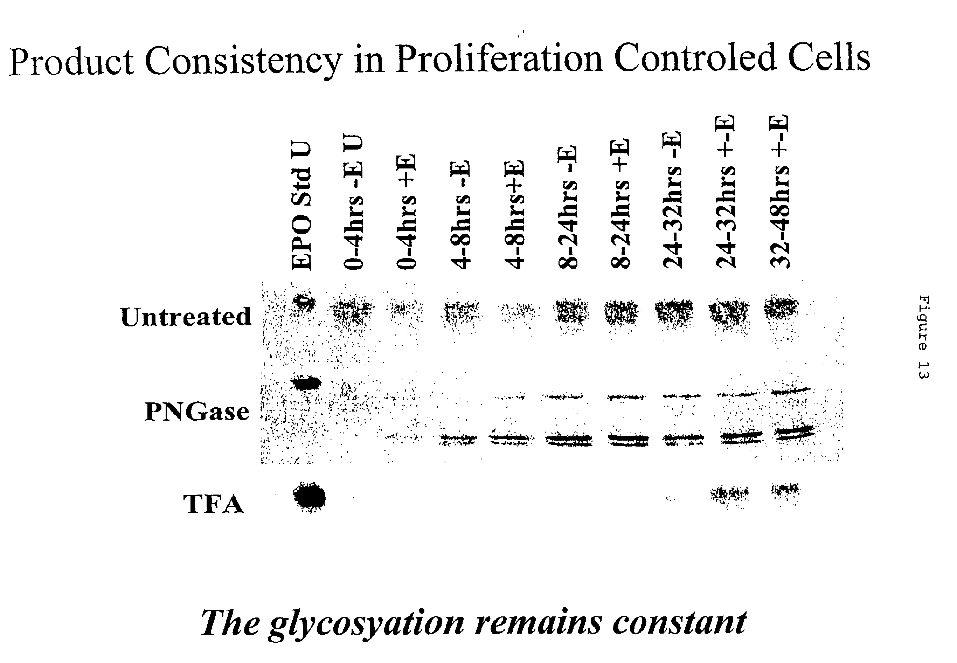

Figure 13 shows an example of high glycoprotein quality in IRF proliferation controlled cells.

### References:

Kirchhoff, S., Kröger, A., Cruz, H., Tummler, M., Schaper, F., Köster, M., Hauser, H. (1996). Regulation of cell growth by IRF-1 in BHK-21 cells. Cytotechnology 22, 147-156.

Colbère-Garapin, F., Horodniceanu, F., Khourilsky, P. and Garapin, A. C. 1981. A new dominant hybrid selection marker for higher eucaryotic cells. J. Mol. Biol. 150: 1-13.

Fussenegger, M., Bailey, J., Hauser, H., and Mueller, P. P. 1998a. Genetic Optimization of Recombinant Protein Production by Mammalian Cells. Trends in Biotechnology, in press

Fussenegger, M., Mazur, X. and Bailey, J. E. 1997. A novel cytostatic process enhances the productivity of Chinese hamster ovary cell. Biotechnol. Bioeng. 55: 927-938

Fussenegger, M., Schlatter, S., Dätwyler, D., Mazur, X. and Bailey, J. E. 1998b. Controlled proliferation by multigene metabolic engineering enhances the productivity of chinese hamster ovary cells. Nat. Biotechnol. 16: 468-472.

Hauser, H. 1997. Heterologous gene expression in mammlian cells, pp. 3-32. In: Hauser, H. and Wagner, R., (eds.), Mammalian Cell Biotechnology in Protein Production, W. DeGruyter, New York.

Kim, Y. H., Iida, T., Fujita, T., Terada, S., Kitayama, A., Ueda, H., Prochownik, E. V. and Suzuki, E. 1998. Establishment of an apoptosis-resistant and growth-controllable cell line by transfecting with inducible antisense c-Jun gene. Biotechnol. Bioeng. 58: 65-72.

Kirchhoff, S., Kröger, A., Cruz, H., Tümmler, M., Schaper, F., Köster, M. and Hauser, H. 1996. Regulation of cell growth by IRF-1 in BHK-21 cells. Cytotechnology 22: 147-156.

Kirchhoff, S., Schaper, F. and Hauser, H. 1993. Interferon regulatory factor 1 (IRF-1) mediates cell growth inhibition by transactivation of downstream target genes. Nucl. Acids Res. 21: 2881-2889.

Koster, M., Kirchhoff, S., Schaper, F. and Hauser, H. 1995. Proliferation control of mammlian cells by the tumor suppressor IRF-1. Cytotechnology 18: 67-75.

Mazur, X., Fussenegger, M., Renner, W.A. and Bailey, J. E. 1998. Higher productivity of growth-arrested chinese hamster ovary cells expressing the cyclin-dependent kinase inhibitor p27. Biotechnol. Prog. 14: 705-713.

Fussenegger, M., Mazur, X. and Bailey, J. E. (1997) A novel cytostatic process enhances the productivity of Chinese hamster ovary cells, Biotechnol. Bioeng. 55, 927-938.

Fussenegger, M., Schlatter, S., Dätwyler, D., Mazur, X. and Bailey, J. E. (1998) Controlled proliferation by multigene metabolic engineering enhances the productivity of Chinese hamster ovary cells, Nat. Biotechnol.16, 468-472.

Fussenegger, M., Bailey, J., Hauser, H. and Mueller, P.P. (1999) Genetic Optimization of Recombinant Protein Production by Mammalian Cells, TIBTECH 17, 43-50.

Hauser, H.: Heterologous expression of genes in mammalian cells. (1997) In: Mammalian Cell Biotechnology in Protein Production. (H. Hauser and R. Wagner, eds.) De Gruyter, Berlin, pp. 1-32.

Kim, Y.H., Iida, T., Fujita, T., Terada, S., Kitayama, A., Ueda, H., Prochownik, E.V. and Suzuki, E. (1998) Establishment of an apoptosis-resistant and growth-controllable cell line by transfecting with inducible antisense c-Jun gene, Biotechnol. Bioeng. 58, 65-72.

Kirchhoff, S., Schaper, F. and Hauser, H. (1993) Interferon regulatory factor 1 (IRF-1) mediates cell growth inhibition by transactivation of downstream target genes, Nucleic Acids Res. 21, 2881- 2889.

Kirchhoff, S., Koromilas, A. E., Schaper, F., Grashoff, M., Sonenberg, N. and Hauser, H. (1995) IRF-1 induced cell growth inhibition and interferon induction requires the activity of the protein kinase PKR, Oncogene 11, 439-445.

Kirchhoff, S., Kroger, A., Cruz, H., Tümmler, M., Schaper, F., Köster, M. and Hauser, H. (1996) Regulation of cell growth by IRF-1 in BHK-21 cells, Cytotechnology 22, 147-156.

Kirchhoff, S., Schaper, F., Oumard, A. and Hauser, H. (1998) In vivo formation of IRF-1 homodimers, Biochimie, in press.

Kirchhoff, S. and Hauser, H. (1999) Cooperative activity between HER oncogenes and the tumor suppressor of IRF-1 results in apoptosis, Oncogene, in press.

Mazur, X., Fussenegger, M., Renner, W.A. and Bailey, J.E. (1998) Higher productivity of growth-arrested Chinese hamster ovary cells expressing the cyclin-dependent kinase inhibitor p27, Biotechnol. Prog. 14, 705-713.

Mitchell, C.A., Beall, J.A., Wells, J.R., Gray, P.P. (1991) Growth and protein production kinetics of a murine myeloma cell line transfected with the human growth hormone gene, Cytotechnology 5, 2232-31.

Miyamoto, M., Fujita, T., Kimura, Y., Maruyama, M., Harada, H., Sudo, Y., Miyata, T. and Taniguchi, T. (1988) Regulated expression of a gene encoding a nuclear factor, IRF-1, that specifically binds to IFN- gene regulatory elements, Cell 54, 903-913.

Mueller, P. P., Kirchhoff, S. and Hauser, H. (1998) in New Developments and New Applications in Animal Cell Technology (Merten, O.W., Perrin, P. and Griffiths, J.B., eds) Kluwer Academic Publishers, pp. 209-213.

Mueller, P.P., Schlenke, P., Nimtz, M., Conradt, H.S. and Hauser, H. (1999).Recombinant glycoprotein product quality in proliferation controlled BHK-21 cells. Submitted.

Arelt, P., Morelle, D., Ausmeier, M., Fitzek, M. and Hauser, H. (1988) Vectors for efficient expression in mammalian fibroblastoid, myeloid, and lymphoid cells via transfection or infection. Gene 68, 213-219.

Boldicke T, Haase B, Bocher M, Lindenmaier W (1995) Human monoclonal antibodies to cytomegalovirus. Characterization and recombinant expression of a glycoprotein-B-specific antibody. Eur J Biochem 234: 397-405.

Fussenegger, M., Bailey, J. E., Hauser, H. and Müller, P. P (1999). Genetic optimization of recombinant glycoprotein production by mammalian cells. Trends in Biotechnology, 17:35-42

Kirchhoff, S., Kroger, A., Cruz, H., Tümmler, M., Schaper, F., Köster, M., Hauser, H. (1996). Regulation of cell growth by IRF-1 in BHK-21 cells. Cytotechnology 22, 147-156.

Kirchhoff, S. and Hauser, H. (1999). Cooperative activity between HER oncogenes and the tumor suppressor IRF-1 results in apoptosis. Oncogene, in press.

Dirks, W., Schaper, F., Kirchhoff, S., Morelle, C. and Hauser, H. (1994) A multifunctional vector family for gene expression in ,mammalian cells. Gene 149, 387-388

Dirks , W., Wirth, M. and Hauser, H. 1993. Dicistronic transcription units for gene expression in mammalian cells. Gene 128: 247-249.

Kirchhoff, S., Kröger, A., Cruz, H., Tümmler, M., Schaper, F., Köster, M. and Hauser, H. 1996. Regulation of cell growth by IRF-1 in BHK-21 cells. Cytotechnology 22: 147-156.

Kirchhoff, S., Schaper, F. and Hauser, H. 1993. Interferon regulatory factor 1 (IRF-1) mediates cell growth inhibition by transactivation of downstream target genes. Nucl. Acids Res. 21: 2881-2889.

Kirchhoff, S., Koromilas, A., Schaper, F., Grashoff, M., Sonenberg, N. and Hauser, H. 1995. IRF-1 induced cell growth inhibition and interferon induction requires the activity of the protein kinase PKR. Oncogene 11: 439-445.

Köster, M., Kirchhoff, S., Schaper, F. and Hauser, H. 1995. Proliferation control of mammlian cells by the tumor suppressor IRF-1. Cytotechnology 18: 67-75.

Sambrook, J., Fritsch, E. F. and Maniatis T. 1989. Molecular cloning: a laboratory manual, Cold Spring Harbor Laboratory, Cold Spring Harbor, NY.

Schlenke, P., Grabenhorst, E., Nimtz, M. and Conradt, H. S. 1998. Construction and characterization of stably transfected BHK-21 cells with human-type sialylation characteristics. Cytotechnology, in press.

Sonenberg, N. 1990. Poliovirus translation. Curr. Top. Microbiol. Immunol. 161: 23-47.

Tanaka, N., Ishihara, M., Lamphier, M. S., Nozawa, H., Matsuyama, T., Mak, T. W., Aizawa, S., Tokino, T., Oren, M., Taniguchi, T. 1996. Cooperation of the tumor suppressors IRF-1 and p53 in response to DNA damage. Nature 382: 816- 818.

Vara, J. A., Portela, A., Ortin, J. and Jiménez, A. 1986. Expression in mammalian cells of a gene from Streptomyces alboniger conferring puromycin resistance. Nucl. Acids Res. 14: 4617-4624.

Wirth, M., Bode, J., Zettlmeissl, G. and Hauser, H. 1988. Isolation of overproducing recombinant mammalian cell lines by a fast and simple selection procedure. Gene 73: 419-426.

## Claims

1. Promoter-transactivator system for inducible high-level mammalian gene expression with the option of cell growth control comprising
(a) a promoter construct (IRFE promoter) having the general structure: 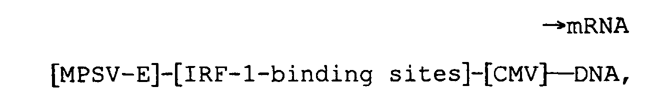 wherein 
MPSV-E means MPSV enhancer repeats of the sequence:
GCTAGCTTAAGTAACGCCATTTTGCAAGGCATGGAAAAATACATAACTGAGAATAGAGAAG
TTCAGATCAAGGTCAGGAACAGAGAAACAGGAGAATATGGGCCAAACAGGATATCTGTGGTA
AGCAGTTCCTGCCCCGCTCAGGGCCAAGAACAGTTGGAACAGGAGAATTGGGCCAAACAGGA
TATCTGTGGTAAGCAGTTCCTGCCCCGCTCAGGGCCAAGAACAGATGGTCCCCAGATGCGGT
CCCGCCCTCAGCAGTTTCTAGA,
 or isofunctional variants thereof obtained by substitution, insertion or deletion of one or more nucleotides,
IRF-1-binding sites means the sequence: 
GATCCCTTCTCGGGAAATGGAAACTGAAAATCAGATCCCTTCTCGGGAAATGGAAACTGAAA
ATCAGATC,
 or isofunctional variants thereof obtained by substitution, insertion or deletion of one or more nucleotides, and
CMV means a minimal promoter of the sequence: 
TGGCGTGTACGGTGGGAGGCCTATATAAGCAGAGCTCGTTTAGTGAACCGTCAAACCGTCAA
ACCGCGGAAGCT,
 or isofunctional variants therof obtained by substitution, insertion or deletion of one or more nucleotides,
and
(b) a transactivator construct coding for a fusion protein comprising IRF-1 and the estrogen receptor.

2. Expression vector(s) comprising a promoter construct and/or a transactivator construct according to claim 1.

3. Mammalian cells transfected or transformed with an expression vector(s) according to claim 2.

4. Process for inducible high-level mammalian gene expression with the option of cell growth control comprising the steps of
(a) transfecting or transforming mammalian cells with an expression vector or expression vectors, respectively, according to claim 2,
(b) culturing said mammalian cells, or transfected or transformed mammalian cells according to claim 3, in a suitable medium, and,
(c) optionally, controlling the growth of said mammalian cells by varying the concentration and the duration of exposure to estradiol in the medium.
